# EUROPEAN PATENT APPLICATION

(11) **EP 1 273 615 A1**
(43) Date of publication of application: **08.01.2003**
(21) Application number: 01948993.9
(22) Date of filing: 01.02.2001
(51) Int. Cl.: C08J 9/28, A61L 15/22, A61L 15/62, A61L 27/14, A61L 27/56, A61L 27/60

(54) **SPONGY MOLDING COMPRISING WATER-SOLUBLE POLYMERIC MATERIAL AND METHOD OF CONTROLLING PORES THEREOF**

(30) Priority: 03.02.2000 JP 2000026774
(71) Applicant: MENICON CO., LTD., Nagoya-shi, Aichi 460-0006 (JP)
(72) Inventor: SUGIE, Toshimasa, c/o MENICON CO., LTD., Kasugai-shi, Aichi 487-0032 (JP); YANAGAWA, Hiroaki, c/o MENICON CO., LTD., Kasugai-shi, Aichi 487-0032 (JP); BABA, Yuji, c/o MENICON CO., LTD., Kasugai-shi, Aichi 487-0032 (JP)
(74) Representative: Leson, Thomas Johannes Alois, Dipl.-Ing.
(86) International application number: JP0100698
(87) International publication number: WO01057121

(57) **Abstract**

A method for controlling pores of a spongy molding comprising a water-soluble polymeric material, which comprises a prefreezing step characterized in that a solution or a gel of the water-soluble material is prefreezed by cooling an interfacial side of the solution or the gel and air layer to induce a temperature slope in parallel with a direction of thickness within the solution or the gel, and a step of lyophilizing a solution or a gel of the water-soluble material prefreezed in according to the prefreezing step. Using the method for controlling pores, a spongy molding having a porous form enabling easily physical connection with the outside and the inside of sponge.

## Description

### TECHNICAL FIELD

The present invention relates to a spongy molding comprising a water-soluble polymeric material and a method for controlling pores thereof. More particularly, present invention relates to a method for controlling pores which comprises by carrying out a prefreezing step in the specific cooling direction and inducing a temperature slope, and a spongy molding prepared by the method, wherein vertically directional pores are formed within the sponge and pores exist in the surface, and which can be suitably used as a wound dressing, a graft for wound and a cultured skin matrix.

### BACKGROUND ART

Conventionally, a molding comprising a water-soluble polymeric material is used as a wound dressing and a graft for wound (especially a graft for dermal defect) which can be applied to a skin defect such as a burn or a bedsore, and thereby promotes healing it, and a cultured skin matrix. As the form thereof, sponge, nonwoven, film, and the like are chosen.

The wound dressing and the graft for wound are considered that a form thereof influences wound healing. For example, the graft for dermal defect is a spongy molding which mainly consists of collagen, and it is the aim that the defective skin tissue is reconstructed by making blood vessels and fibroblasts infiltrate into the sponge from circumference of wound. It is considered that the size, the continuity and the orientation of pores in the sponge interact with the infiltration of the blood vessels and the fibroblasts.

However, since it is the discontinuous independent pores which are formed in the wound dressing and the graft for wound already taken out to the market, it cannot expect that the blood vessels and the fibroblasts infiltrate over the wide range inside sponge.

As a raw material of a cultured skin matrix, tissue-derived biomaterial, such as collagen, atelocollagen, gelatin, a chitin, chitosan, a fibronectin, a laminin or a hyaluronic acid is often used, and investigation thereof is advanced. Generally, the cultured skin comprises of a matrix thereof and fibroblasts, keratinocytes derived from skin and so on. If the cultured skin is applied to a skin defect, it is said that the humoral factor produced by the above-mentioned fibroblasts, the keratinocytes or the like enhances a migration and a proliferation of fibroblasts, keratinocytes and vascular endothelial cells from the edge of the wound, and makes the skin reconstruct. The cultured skin can be prepared by dropping suspension of the above-mentioned fibroblasts or keratinocytes on the matrix, and by cultivating it with the suspension adhering during a fixed period. When the cultured skin matrix is a spongy molding, if the diameter of pores at the surface of the sponge is constant and future the form of internal pores is constant, the dropped cell can be uniformly adhered to the matrix. Therefore, it is expected that the resulting cultured skin has a constant ability for wound healing.

However, since in the conventional cultured skin matrix, the size of the internal pores is nonuniformity and the pores do not oriented, a uniformal adhesion of the dropped cell and a constant ability for wound healing cannot be expected. Furthermore, since the pores of the conventional cultured skin matrix is independent pores, it is considered that the humoral factor produced from the fibroblasts and the keratinocytes inside the cultured skin on applying to a defect cannot easily reach to tissue or cells in and around wound.

Conventionally, for example, the followings are proposed as spongy material applying to the defect and the process for preparing the same.

In Japanese Unexamined Patent Publications No. 167331/1987, a chitosan sponge having at least 80 % of porosities, which comprises chitosan dissolving in the diluted acetic acid aqueous solution is disclosed. Although the chitosan sponge can be obtained by lyophilizing without detriment of porosity, a condition of the freeze and a forming direction of the pores are not clear.

Japanese Unexamined Patent Publications No. 4629/1989 discloses a collagen sponge in which a forming direction of fine pores is in parallel with the direction of thickness of sample, and a diameter of the pores is uniformed. However, since a detergent is used in order to control the fine pores of the collagen sponge, the removal step is required, and thereby the operation becomes vexatious complication. A denaturation of protein by the detergent is concerned. Furthermore, although the collagen sponge can be obtained by a freezing method, a freezing temperature, a freezing rate and the like are not clear.

In Japanese Unexamined Patent Publications No. 208332/1990 discloses a polymeric porous object having a structure such that a continuous pore being a communicating vacuole reaches to internal part from the surface, actual vertically. However, there is no any concrete description as to a forming method of the continuous pores and the surface state of a polymeric porous object is not clear.

Japanese Unexamined Patent Publications No. 261838/1990 discloses a porous chitosan material. But the pores are continuous pores constructing a complex three dimensional network.

In Japanese Unexamined Patent Publications No. 332561/1992 discloses a sheet of collagen sponge set up penetrating pores, mechanically. However, since the collagen sponge sheet is mechanically set up penetrating pores, most pores may be closed if the sponge permeates with a culture medium or the exudation liquid from the defect. Herewith, a cellular infiltration from the edge of the defect and cell infiltration on dropping a cell suspension may be adversely affected.

Although the different spongy materials and the preparing process the same is proposed, it is considered that the following conditions are needed for the porous form of spongy molding used as the wound dressing, the graft for wound (especially the graft for dermal defect) and the cultured skin matrix.
(1) The pore has a form with the easy physical connection with the outside and the inside of sponge. Preferably, pores have penetrated into the sponge with orientation.
(2) In order to allow a cell to easily infiltrate inside of sponge, pores which has a constant size on the surface of sponge should be formed.
(3) If it is necessity, in order to prevent the outflow of a cell or body fluid, a pore may hardly be formed in another surface (the surface of opposite to the surface in which the pores having a constant size was formed in the above-mentioned (2)), and the surface may mainly be a film-like.

That is, it is thought that the spongy molding which fill the above-mentioned conditions of (1)-(3) relating porous form is ideal as the wound dressing, the graft for wound (especially the graft for dermal defect), and the cultured skin matrix.

Therefore, it is hard to say that each conventional various spongy material is an ideal spongy molding. Additionally, the method of obtaining such ideal spongy molding with ease is not proposed.

The present invention is made in view of the above-mentioned background, and provides a method for controlling pores of a spongy molding which has plurality of pores in parallel with a direction of thickness, and a spongy molding prepared by the method for controlling pores.

### DISCLOSURE OF INVENTION

The present invention relates to the followings:
(1) a method for controlling pores of a spongy molding comprising a water-soluble polymeric material, which comprises
   (a) a prefreezing step characterized in that a solution or a gel of the water-soluble material is prefreezed by cooling a interfacial side of the solution or the gel and air layer to induce a temperature slope in parallel with the direction of thickness within the solution or the gel,
   (b) a step of lyophilizing the solution or the gel of the water-soluble material prefreezed in according to the prefreezing step (a); and
(2) a spongy molding prepared according to the above-mentioned method for controlling pores, which has plurality of vertically directional pores in parallel with the direction of thickness, in which the above-mentioned plurality of vertically directional pores are opened in a surface which was the interface and/or the opposite surface as to the surface which was the interface; and
(3) a method for controlling pores of a spongy molding comprising a water-soluble polymeric material, which comprises
   (a) a prefreezing step characterized in that a solution or a gel of the water-soluble material is prefreezed by cooling a interfacial side of the solution or the gel and air layer prior to other surfaces excluding the interfacial side to induce a temperature slope in parallel with the direction of thickness within the solution or the gel,
   (b) a step of lyophilizing the solution or the gel of the water-soluble material prefreezed in according to the prefreezing step (a); and
(4) a spongy molding prepared according to the above-mentioned method for controlling pores, which has plurality of vertically directional pores in parallel with the direction of thickness, in which the above-mentioned plurality of vertically directional pores are opened in the surface which was the interface and/or the opposite surface as to the surface which was the interface.

### BRIEF DESCRIPTION OF DRAWINGS

Fig. 1 (a) and (b) are each a schematic explanation view showing an embodiment of a method for controlling pores according to the present invention.
Fig. 2 is a schematic explanation view showing an embodiment of a method for controlling pores according to the present invention.
Fig. 3 is a schematic explanation view showing an embodiment of a method for controlling pores according to the present invention.
Fig. 4 is a schematic explanation view showing an embodiment of a method for controlling pores according to the present invention.
Fig. 5 is a schematic explanation view showing an embodiment of a vessel used in a method for controlling pores according to the present invention.
Fig. 6 is a schematic explanation view showing an embodiment of a method for controlling pores according to the present invention.
Fig. 7 is a scanning electron micrograph of a surface of an atelocollagen sponge obtained in Example 1.
Fig. 8 is a scanning electron micrograph of a cross section of an atelocollagen sponge obtained in Example 1.
Fig. 9 is a scanning electron micrograph of a surface of an atelocollagen sponge obtained in Example 2.
Fig. 10 is a scanning electron micrograph of a cross section of an atelocollagen sponge obtained in Example 2.
Fig. 11 is a scanning electron micrograph of a surface of an atelocollagen sponge obtained in Example 3.
Fig. 12 is a scanning electron micrograph of a cross section of an atelocollagen sponge obtained in Example 3.
Fig. 13 is a scanning electron micrograph of a surface of a chitosan/atelocollagen sponge (chitosan : atelocollagen (by weight) = 25 : 75) obtained in Example 4.
Fig. 14 is a scanning electron micrograph of a cross section of a chitosan/atelocollagen sponge (chitosan : atelocollagen (by weight) = 25 : 75) obtained in Example 4.
Fig. 15 is a scanning electron micrograph of a surface of a chitosan/atelocollagen sponge (chitosan : atelocollagen (by weight) = 50 : 50) obtained in Example 5.
Fig. 16 is a scanning electron micrograph of a cross section of a chitosan/atelocollagen sponge (chitosan : atelocollagen (by weight) = 50 : 50) obtained in Example 5.
Fig. 17 is a scanning electron micrograph of a surface of a chitosan/atelocollagen sponge (chitosan : atelocollagen (by weight) = 75 : 25) obtained in Example 6.
Fig. 18 is a scanning electron micrograph of a cross section of a chitosan/atelocollagen sponge (chitosan : atelocollagen (by weight) = 75 : 25) obtained in Example 6.
Fig. 19 is a scanning electron micrograph of a surface of a chitosan sponge obtained in Example 7.
Fig. 20 is a scanning electron micrograph of a cross section of a chitosan sponge obtained in Example 7.
Fig. 21 is a scanning electron micrograph of a surface of an atelocollagen sponge obtained in Comparative Example 1.
Fig. 22 is a scanning electron micrograph of a cross section of an atelocollagen sponge obtained in Comparative Example 1.
Fig. 23 is a scanning electron micrograph of a surface of a chitosan sponge obtained in Comparative Example 2.
Fig. 24 is a scanning electron micrograph of a cross section of a chitosan sponge obtained in Comparative Example 2.
Fig. 25 is a graph showing the temperature history of a gel during the prefreezing in Example 11.
Fig. 26 is a scanning electron micrograph of a surface of an atelocollagen sponge obtained in Example 11.
Fig. 27 is a scanning electron micrograph of a cross section of an atelocollagen sponge obtained in Example 11.
Fig. 28 is a graph showing the temperature history of a gel during the prefreezing in Example 12.
Fig. 29 is a scanning electron micrograph of a surface of an atelocollagen sponge obtained in Example 12.
Fig. 30 is a scanning electron micrograph of a cross section of an atelocollagen sponge obtained in Example 12.
Fig. 31 is a graph showing the temperature history of a gel during the prefreezing in Example 13.
Fig. 32 is a scanning electron micrograph of a surface of an atelocollagen sponge obtained in Example 13.
Fig. 33 is a scanning electron micrograph of a cross section of an atelocollagen sponge obtained in Example 13.
Fig. 34 is a graph showing the temperature history of a gel during the prefreezing in Example 14.
Fig. 35 is a scanning electron micrograph of a surface of an atelocollagen sponge obtained in Example 14.
Fig. 36 is a scanning electron micrograph of a cross section of an atelocollagen sponge obtained in Example 14.

### BEST MODE FOR CARRYING OUT THE INVENTION

A spongy molding prepared by the method for controlling pores of the present invention has an ideal, porous form, wherein plurality of vertically directional pores (in parallel with the direction of thickness of spongy molding) are formed within the spongy molding, and pores having a constant size are formed in one surface (surface which was the interface of a solution or a gel of a water-soluble polymeric material being the material of the spongy molding). Additionally, the spongy molding of the present invention, if necessary, may be a spongy molding such that a pore is hardly formed in the opposite surface to the surface in which pores having the constant size are formed, and that the opposite surface is substantially closed.

In the present invention, a lyophilizational technique is employed in order to form the sponge comprising a water-soluble polymeric material. Generally, it is known that a product by lyophilizing have advantages such that it prevents bacteria and fungus from proliferating, and that it improves preservation stability.

The spongy molding using a water-soluble polymeric material as a raw material can be prepared using the lyophilizational technique in following order.
(1) The water-soluble material is dissolved in water, acidic aqueous solution, alkaline aqueous solution or organic solvent.
(2) The solution of the water-soluble polymeric material obtained in the above (1) is poured into a plastic case, a stainless-steel case or the like. Alternatively, it is poured on the plastic flat plate or the stainless-steel flat plate. If necessary, the solution may be gelated. When the solution is gelated, the obtained gel may be removed to the flat plate.
(3) A prefreezing is carried out by infiltrating with liquid nitrogen or putting into a freezer or a lyophilizer.
(4) A lyophilization is carried out.

Pores existing in the spongy molding are parts in which the solvent was sublimed by the lyophilization. The morphology of the pore is generally determined by how the solvent and the solute were phase-separated at the time of the prefreezing. That is to say, the control of porous form in the spongy molding is realizable by controlling the form of phase separation arising at the time of prefreezing, concretely by controlling cooling temperature and cooling direction of the solution or the gel.

Next, the principle of control of a porous form is explained below.

When the solution or the gel of the water-soluble polymeric material is cooled down, in case of water as the solvent, ice crystal is formed. The ice crystal is generally formed by firstly generating a nucleus, and then the nucleus growing using near water. For example, when the solution or the gel of the water-soluble polymeric material represented by chitosan aqueous solution, collagen aqueous solution, collagen gel, chitosan/collagen gel, chitosan/collagen aqueous solution and the like is cooled from one direction, firstly, in near the interface a phase separation occurs to arise a solvent rich region and a solute rich region, and the water existing in the solvent rich region become the crystal nucleus. Thereafter, the cooling develops toward a deep layer and the same phase separation occurs continuously. At that time, the crystal nucleus of ice produced at the interface grows toward a deep layer using water separated in the deep layer. Consequently, the continuously vertical ice crystal is formed. However, a generation and its growth of a crystal nucleus of ice are the phenomenon which may happen under near the eutectic temperature of the solution or the gel of the water-soluble polymeric material (it is the temperature at which two or more kinds of crystals deposit simultaneously from the solution or the gel, and has a certain range of temperature), the phenomenon which hardly happen at either the temperature beyond the eutectic temperature or the temperature not more than the eutectic point.

It is thought that the vertically directional ice crystal cannot be formed at the time of cooling if cooling rate is both too quick and too slow. Moreover, if cold air is allowed to reach to the inside of the solution or the gel of the water-soluble polymeric material without controlling the cooling direction, it is thought that it is difficult to form the vertical ice crystal.

First, when cooling rate is too quick, even if crystal nucleus of ice can be formed in the interface of this solution or gel by introducing cold air from an interfacial side, the depths are cooled before it fully grows, and thereby new crystal nucleus of ice is formed there. Consequently, a spherical, relatively small crystal is easy to be formed, without forming the vertical crystal of ice. That is, independent pores are easy to be formed when it molds to sponge.

On the contrary, when cooling rate is too slow, since the crystal nucleus formed in the interface of the solution or the gel has sufficient time for the growth, and therefore, it has the tendency for the big ice crystal to be formed in some places. That is, when it molds to sponge, the set of an independent hole having a certain orientation serves as a form distributed over some places.

Moreover, when the solution or the gel of the water-soluble polymeric material is cooled without controlling the cooling direction, it cools toward the inside of the solution or the gel from various directions. Since cold air is introduced from six directions when the solution or the gel is assumed to be, for example, a hexahedron, a crystal nucleus of ice generates in each interfacial side. And since it is thought that it cools toward the center of the solution or the gel from each side, an ice crystal grows toward the center similarly. Therefore, in the inside of the molded sponge, the pores generally orientated toward the center are formed.

Then, especially, in order to make the vertically directional pores form in a spongy molding the following is founded due to attention paid cooling direction of the solution or the gel at the time of a prefreezing. When the solution or the gel was put into a container, was cooled by hitting cold air from the interfacial side, especially, prior to other interfacial side excluding the interfacial side, for example, when a solvent was water, an ice crystal may be generally grown toward a lower part (opposite side as to an interface).

Namely, after performing a prefreezing by the method of cooling an interfacial side of the solution or the gel with air layer like the above, by molding it into sponge with the lyophilization, the spongy molding having a vertically directional pores in which the pores having the constant size are formed in the surface (surface which was the interface of the solution or the gel of the water-soluble polymeric material which is the material of the spongy molding) and/or the opposite surface as to the surface can be obtained. The spongy molding is very ideal as the wound dressing, the graft for wound (especially the graft for dermal defect) and the cultured skin matrix.

As a water-soluble polymeric material used in a method for controlling pores of the present invention, examples are polyacrylamide, polyvinyl alcohol, polyethylene glycol, water-soluble cellulose derivative, and tissue-derived biomaterials such as collagen, atelocollagen, hyaluronic acid, gelatin, chitin and chitosan. It is desirable that at least one sort of these are contained in the solution or the gel of the water-soluble polymeric material. Among the above-mentioned water-soluble polymeric materials, at least one sort of tissue-derived biomaterials selected from the group consisting of collagen, atelocollagen, hyaluronic acid, gelatin, chitin and chitosan is preferable. Also among this tissue-derived biomaterials, collagen, atelocollagen and chitosan, especially atelocollagen and/or chitosan are preferable from a viewpoint of molding.

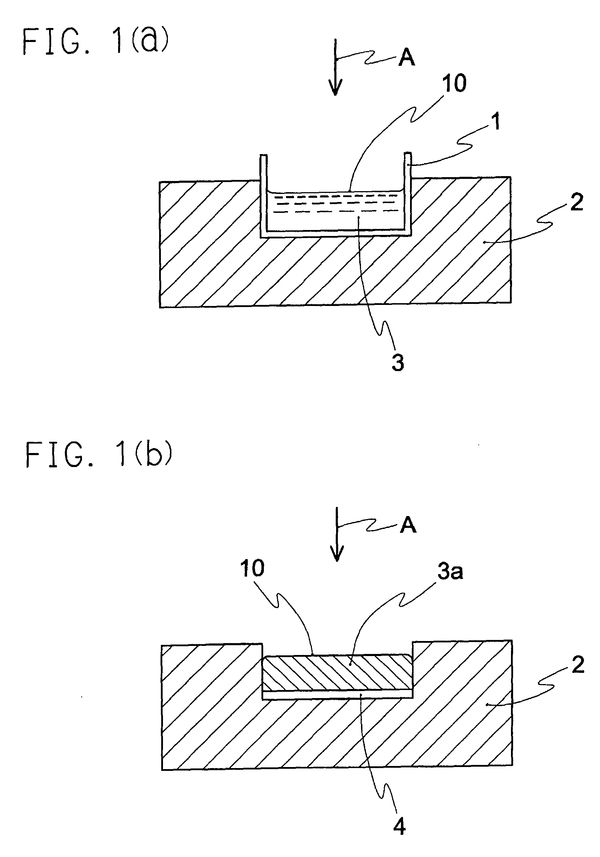

The method of cooling the solution or the gel of the water-soluble polymeric material does not have particularly limitation as long as it is a method of cooling an interfacial side of the solution or the gel and air layer, especially the method of cooling the interfacial side prior to other faces excluding the interfacial side. For example, the method of cooling from the interfacial side in state where other faces excluding the interfacial side are covered with a heat insulation object is preferable. In the present invention, for example, the method shown in Figs. 1-6 can be employed.

Each of Figs. 1 (a) and (b) is a schematic explanation view showing one embodiment of the method for controlling pores of the present invention. The interface 10 of the solution or the gel 3 can be cooled in the state where it was insulated with the heat insulation object 2 except for the opening of the container 1 containing the solution or the gel 3 of the water-soluble polymeric material as shown in Fig. 1 (a), by putting it into an ultra-deep freezer, hitting cold air to the interface 10 from the upper part of the opening of this container 1 in the direction of the arrow A prior to other faces excluding the interface 10.

The above-mentioned opening may be the whole area of the upper part of the container, and in the case of the container with a lid, it may be provided in a part of the lid or it may be provided in the side of the container so that it may not be dipped with the solution or the gel of the water-soluble polymeric material.

Furthermore, as shown in Fig. 1 (b), when a water-soluble polymeric material is a gel, the interface 10 can be cooled by pouring the gel 3a of the water-soluble polymeric material on the plate 4 made from plastic or stainless steel, changing into the state where it was insulated with the heat insulation object 2 except for the upper part, and introducing cold air in the direction of the arrow A from the upper part prior to other interfacial side excluding the interface 10 of the gel 3a.

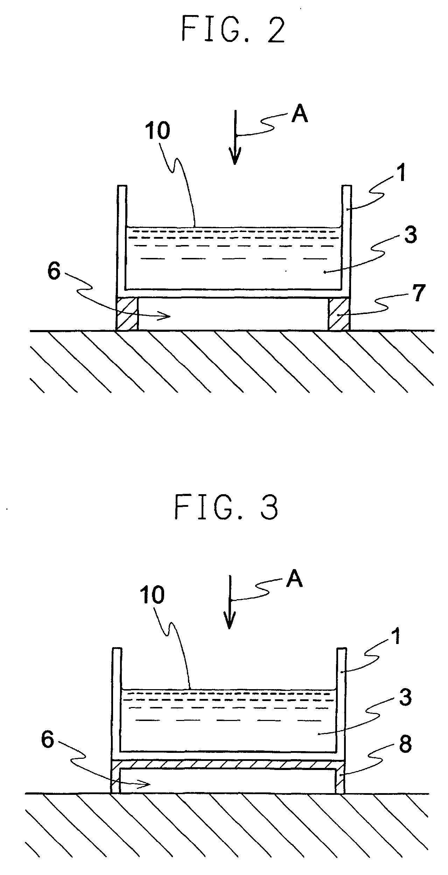

Moreover, a container in which the air layer 6 is made to exist under the bottom of the container 1 by placing the container 1 on the component 7 as shown in a schematic explanation view showing one embodiment of the method for controlling pores of the present invention of Fig. 2, or a container in which the air layer 6 is made to exist under the bottom of the container 1 by lying the empty case 8 prone and placing the container thereon as shown in a schematic explanation view showing one embodiment of the method for controlling pores of the present invention of Fig. 3 can be employed as a container to contain the solution or the gel of the water-soluble polymeric material. The interface 10 can be cooled by introducing cold air from the upper part of the opening of this container 1 in the direction of the arrow A, prior to other interfacial sides excluding the interface 10 of the solution or the gel 3 by putting the container into an ultra-deep freezer. Herein, as material of a component 7 and a case 8, there is plastic or the like. A material which can support the container 1 is sufficient.

In the method for controlling pores shown in the above-mentioned figures 2 and 3, since the air layer 6 exists under the bottom of the container 1 containing the solution or the gel 3 of the water-soluble polymeric material, the air layer 6 also carries out the role of a heat insulation object.

Moreover, the solution or the gel of the water-soluble polymeric material can be cooled by contacting liquid nitrogen to the interface of the solution or the gel, or by placing the interface of the solution or the gel under nitrogen gas atmosphere. Furthermore, by cooling the piece of a metal provided so that the interface of the solution or the gel of the water-soluble polymeric material might be made to contact directly or indirectly, it may be cooled in the state where the cooling direction is retained. Furthermore, by contacting directly or indirectly a piece of a metal, a polymer gel or the like which was cooled beforehand to the interface of the solution or the gel of the water-soluble polymeric material, it may be cooled in the state where the directivity of cooling is retained.

In addition, herein "it being made to contact indirectly" means the state being "through an air layer", for example, "the piece of a metal is indirectly contacted to an interface" means the state that an air layer exists between the piece of a metal and the interface, and that a transfer of heat is performed through this air layer from the piece of a metal to the interface.

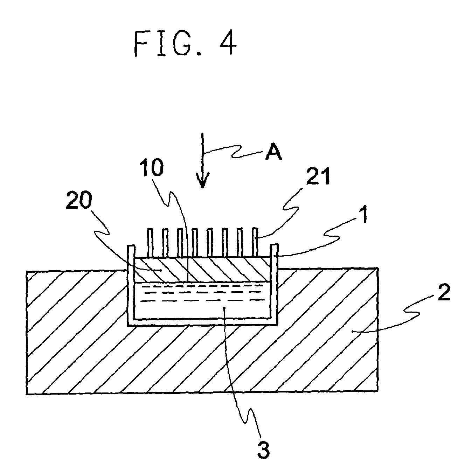

Furthermore, for example, when the piece of a metal 20 in which many fins 21 for cooling were provided is used in order to increase a cooling side as shown in a schematic explanation view showing one embodiment of the method for controlling pores of the present invention of Fig. 4, there is the following advantage. Namely, after cooling the piece of a metal 20 beforehand, by contacting this piece of a metal 20 to the interface 10 of the solution or the gel 3 of the a water-soluble polymeric material, directly or indirectly, the solution or the gel 3 of the water-soluble polymeric material can be cooled. After contacting the piece of a metal 20 to the interface 10 of the solution or the gel 3 of the water-soluble polymeric material, the interface 10 of the solution or the gel 3 of the water-soluble polymeric material can be indirectly cooled through the piece of a metal 20, and the fin 21 for cooling by introducing cold air from the upper part of the opening of the container 1 in the direction of the arrow A. In this case, other part excluding the opening of the container 1 may be changed into the state where it was insulated with the heat insulation object 2, if needed.

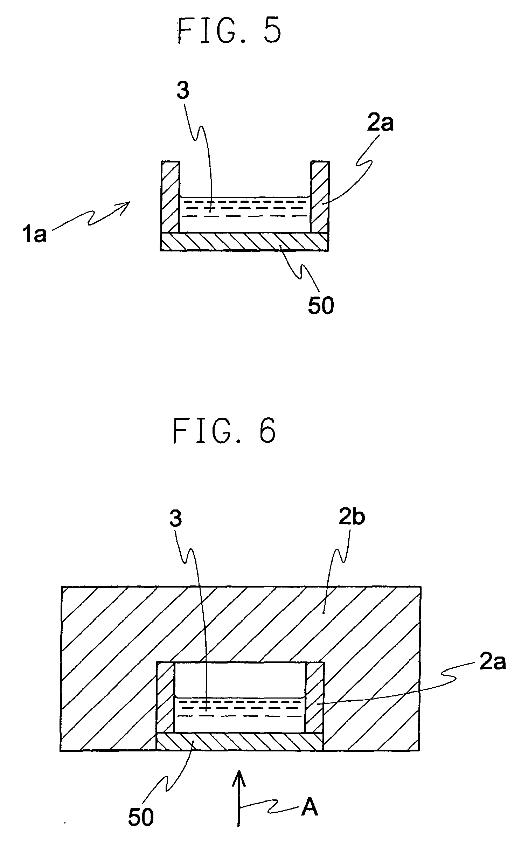

Moreover, a schematic explanation view is shown in Fig. 5 as one embodiment of the container used for the method for controlling pores of the present invention. As shown in Fig. 5, the solution or the gel 3 of the water-soluble polymeric material can be poured into the container 1a which equipped the side with polystyrene foam 2a, and equipped the bottom with the stainless steel plate or the plastic plate 50. Using the container 1a, as shown in a schematic explanation view showing one embodiment of the method for controlling pores of the present invention of Fig. 6, the opening and the side of the container 1a may be insulated with the heat insulation frame 2b which the hole capable insert the container 1a is opened in the lower part, and the stainless steel plate or the plastic plate 50 at the bottom may be cooled from the direction of the arrow A.

In the present invention, the cooling temperature on prefreezing the solution or the gel of the water-soluble polymeric material may be temperature at which the solution or the gel can freeze, but not particularly limitation.

In the present invention, it is necessary that temperature slope should be induced in parallel with the direction of thickness inside of the solution or the gel of the water-soluble polymeric material in order to make a vertically directional pore form within a spongy molding. At this time, when the solution or the gel becomes considering water or an aqueous solution as a solvent, the cooling rate of the solution or the gel is desirably at least ±0.00°C/min. Since it has sufficient time for the crystal nucleus formed in the interface of the solution or the gel to grow when the cooling rate is too slow, the big crystal of ice is formed in some places and thereby it becomes the form where the set of an independent pore having a certain orientation was distributed over some places on molding it into sponge. Moreover, when cooling rate is too quick, even if it can form a crystal nucleus of ice in the interface of the solution or the gel, before it fully grew, since new crystal nucleus of ice is to be formed due to cooling the deep part, a spherical, relatively small crystal is easy to be formed and thereby the independent pore can be easily formed on molding it into sponge. Therefore, cooling rate is desirably at least -100°C/min, preferably at least -40°C/min.

A heat insulation object used for present invention can be employed any object as long as the interfacial side of a solution or a gel of a water-soluble polymeric material with air layer allowed to cool prior to other interfacial side excluding the interfacial side. Such Examples are foam glass, firing concrete, polystyrene foam, foaming urethane, plastics, metal, ceramics, gases such as air, and the like. These heat insulation objects can also be used in the combination.

The diameter and length of a vertically directional pore which are formed by the method for controlling pores of the present invention can be made into discretional diameters and length by adjusting cooling rate, thickness at the time when a solution or a gel is poured, and the like.

In addition, it is preferable that the diameter of a vertically directional pore is about 10-1000 µm in order to prevent fall down of keratinocytes and fibroblasts and so that the keratinocytes, the fibroblasts and vascular endothelial cells may easily infiltrate, but not limited thereto.

Since the vertically directional pore is formed in the inside of it and a pore exists in the surface, the spongy molding of the present invention obtained in this manner can be especially used suitably as a wound dressing, a graft for wound and cultured skin matrix, for example.

The spongy molding comprising a water-soluble polymeric material of the present invention and the method for controlling pores thereof are explained in more detail by means of Examples but the present invention is not limited only to the Examples.

### EXAMPLE 1

### (Preparation of atelocollagen sponge)

About 20 g of 1.0 % atelocollagen/citric acid aqueous solution (derived from the skin of adult pig) was poured into a plastic case having a size of about 100 × 100 × 20 mm, and was gelated in an atmosphere of ammonia gas. Freezing the gel was carried out by inserting the plastic case in the heat insulation frame made from polystyrene foam (having a size of about 200 × 200 × 70 mm, and in the upper part thereof, the hole in which the above-mentioned plastic case can be inserted being open), putting whole heat insulation frame into an ultra-deep freezer (setting temperature: about -150 to -20°C, the same temperature was presupposed also in the following Examples 2-10 and Comparative Examples 1-2.), and then lyophilization was carried out. The obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

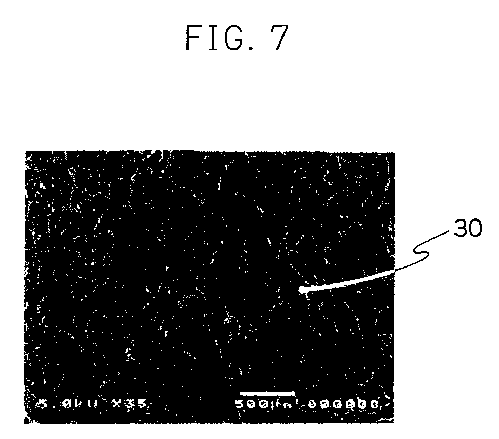

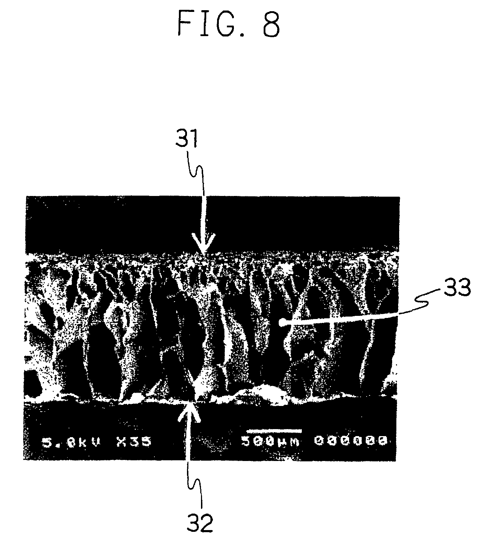

The surface and the cross section of the obtained atelocollagen sponge were observed with the scanning electron microscope (made by JEOL Ltd., JFC-1500, using in the following Examples 2-14 and Comparative Examples 1-2 also). Fig. 7 is the scanning electron micrograph (original magnification × 35) of the surface of the obtained atelocollagen sponge and Fig. 8 is the scanning electron micrograph (original magnification × 35) of the cross section of the obtained atelocollagen sponge.

As shown in Fig. 8, the vertically directional pores 33 penetrated from the surface 31 of sponge to near the rear surface 32 were formed, and pores 30 having a diameter of 200-300 µm were uniformly formed in the surface 31 (refer to Fig. 7).

### EXAMPLE 2

### (Preparation of atelocollagen sponge)

About 20 g of 1.0 % atelocollagen/citric acid aqueous solution (derived from the skin of adult pig) was poured into the plastic case (container 1) having a size of about 100 × 100 × 20 mm, and was gelated in an atmosphere of ammonia gas. As shown in Fig. 2, freezing the gel was carried out by putting that plastic case on a plastic component (component 7), putting it into the ultra-deep freezer and then lyophilization was carried out. The obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

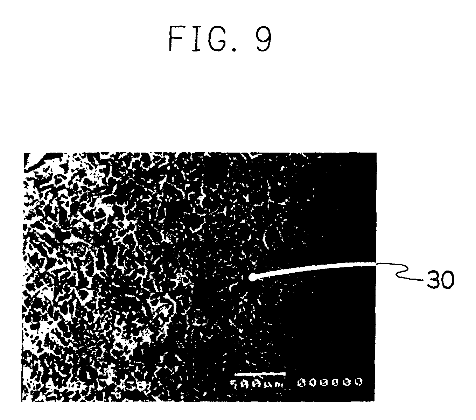

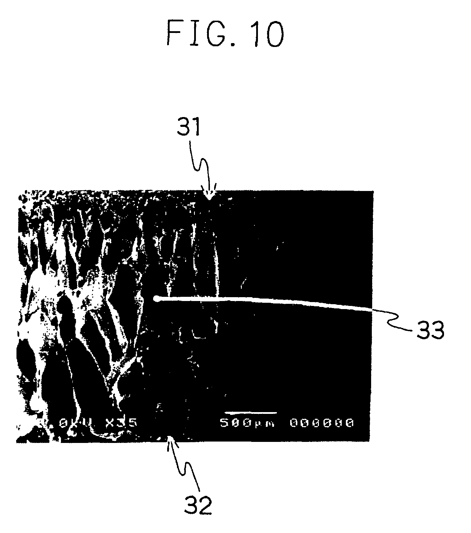

The surface and the cross section of the obtained atelocollagen sponge were observed with the scanning electron microscope. Fig. 9 is the scanning electron micrograph (original magnification × 35) of the surface of the obtained atelocollagen sponge and Fig. 10 is the scanning electron micrograph (original magnification × 35) of the cross section of the obtained atelocollagen sponge.

As shown in Fig. 10, the vertically directional pores 33 penetrated from the surface 31 of sponge to near the rear surface 32 were formed, and the pores 30 having diameter of several 10 µm were uniformly formed in the surface 31 (refer to Fig. 9).

### EXAMPLE 3

### (Preparation of atelocollagen sponge)

About 20 g of 1.0 % atelocollagen/citric acid aqueous solution (derived from the skin of adult pig) was poured into the plastic case (container 1) having the size of about 100 × 100 × 20 mm, and was gelated in an atmosphere of ammonia gas. As shown in Fig. 3, freezing the gel was performed by carrying that plastic case on an empty plastic case (case 8) laid prone, putting it into the ultra-deep freezer and then lyophilization was carried out. The obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

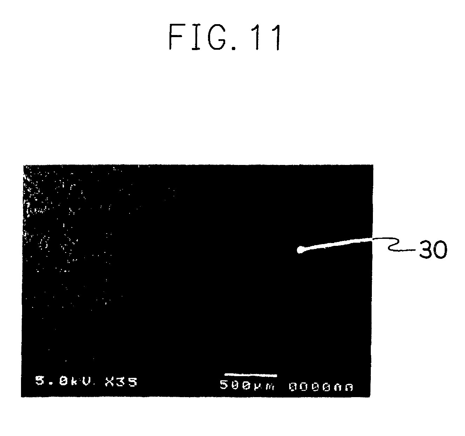

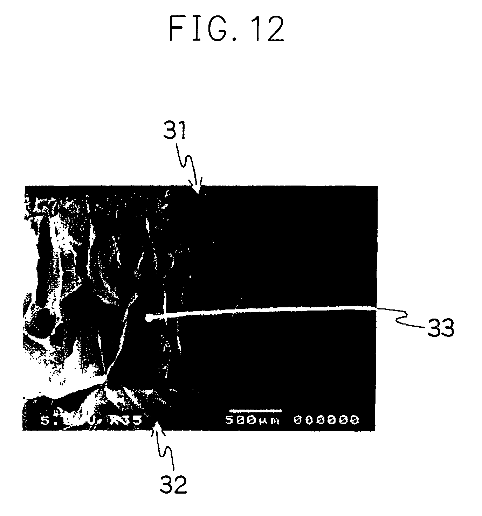

The surface and the cross section of the obtained atelocollagen sponge were observed with the scanning electron microscope). Fig. 11 is the scanning electron micrograph (original magnification × 35) of the surface of the obtained atelocollagen sponge and Fig. 12 is the scanning electron micrograph (original magnification × 35) of the cross section of the obtained atelocollagen sponge.

As shown in Fig. 12, the vertically directional pores 33 penetrated from the surface 31 of sponge to near the rear surface 32 were formed, and the pores 30 having a diameter of several 10 µm were uniformly formed in the surface 31 (refer to Fig. 11).

### EXAMPLE 4

### (Preparation of chitosan solution)

About 1 g of chitosan (derived from crab shell) was added to about 99 g of ultra pure water and was gently stirred at room temperature. With stirring, thereto 1 ml of acetic acid was added and the stirring was continued for about 4 hours at room temperature to obtain a chitosan solution.

### (Preparation of chitosan/atelocollagen sponge)

The above-mentioned chitosan solution in amount of 25 g was combined with 75 g of 1.0 % atelocollagen/citric acid aqueous solution as the same in Example 1. About 20 g of the mixture was poured into a plastic case having a size of about 100 × 100 × 20 mm, and was gelated in an atmosphere of ammonia gas. Freezing the gel was carried out by inserting the plastic case in the heat insulation frame made from polystyrene foam as the same in Example 1, putting whole heat insulation frame into the ultra-deep freezer, and then lyophilization was carried out. The obtained sponge was crosslinked by irradiating ultraviolet ray. This sponge was washed by rinsing in 1 mol/L sodium hydroxide aqueous solution, again, the freeze and the lyophilization were performed to obtain a chitosan/atelocollagen (chitosan : atelocollagen (weight ratio)= 25 : 75) sponge (a spongy molding of the present invention).

### (Observation by scanning electron microscopy)

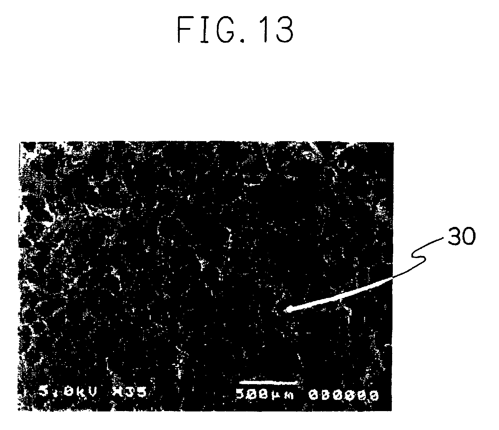

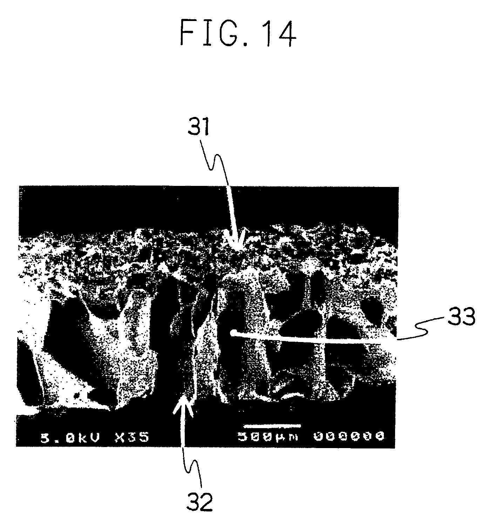

The surface and the cross section of the obtained chitosan/atelocollagen sponge were observed with the scanning electron microscope). Fig. 13 is the scanning electron micrograph (original magnification × 35) of the surface of the obtained chitosan/atelocollagen sponge and Fig. 14 is the scanning electron micrograph (original magnification × 35) of the cross section of the obtained chitosan/atelocollagen sponge.

As shown in Fig. 14, the vertically directional pores 33 penetrated from the surface 31 of sponge to near the rear surface 32 were formed, and the pores 30 having a diameter of 200-300 µm were uniformly formed in the surface 31 (refer to Fig. 13).

### EXAMPLE 5

### (Preparation of chitosan solution)

A chitosan solution was prepared in the same manner as described in Example 4.

### (Preparation of chitosan/atelocollagen sponge)

The above-mentioned chitosan solution in amount of 50 g was combined with 50 g of 1.0 % atelocollagen/citric acid aqueous solution as the same in Example 1. About 20 g of the mixture was poured into a plastic case having a size of about 100 × 100 × 20 mm. Freezing the solution was carried out by inserting the plastic case in the heat insulation frame made from polystyrene foam as the same in Example 1, putting whole heat insulation frame into the ultra-deep freezer, and then lyophilization was carried out. The obtained sponge was crosslinked by irradiating ultraviolet ray. This sponge was washed by rinsing in 1 mol/L sodium hydroxide aqueous solution, again, the freeze and the lyophilization were performed to obtain a chitosan/atelocollagen (chitosan : atelocollagen (weight ratio)= 50 : 50) sponge (a spongy molding of the present invention).

### (Observation by scanning electron microscopy)

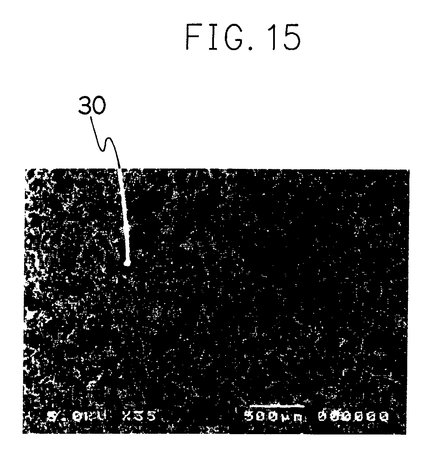

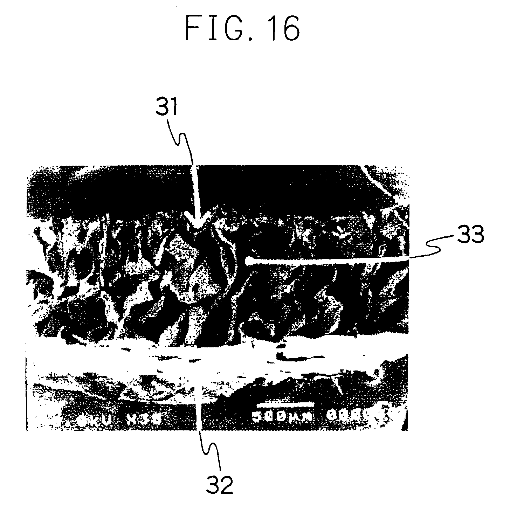

The surface and the cross section of the obtained chitosan/atelocollagen sponge were observed with the scanning electron microscope. Fig. 15 is the scanning electron micrograph (original magnification × 35) of the surface of the obtained chitosan/atelocollagen sponge and Fig. 16 is the scanning electron micrograph (original magnification × 35) of the cross section of the obtained chitosan/atelocollagen sponge.

As shown in Fig. 16, the vertically directional pores 33 penetrated from the surface 31 of sponge to near the rear surface 32 were formed, and the pores 30 having a diameter of 200-300 µm were uniformly formed in the surface 31 (refer to Fig. 15).

### EXAMPLE 6

### (Preparation of chitosan solution)

A chitosan solution was prepared in the same manner as described in Example 4.

### (Preparation of chitosan/atelocollagen sponge)

The above-mentioned chitosan solution in amount of 75 g was combined with 25 g of 1.0 % atelocollagen/citric acid aqueous solution as the same in Example 1. About 20 g of the mixture was poured into a plastic case having a size of about 100 × 100 × 20 mm. Freezing the solution was carried out by inserting the plastic case in the heat insulation frame made from polystyrene foam as the same in Example 1, putting whole heat insulation frame into the ultra-deep freezer, and then lyophilization was carried out. The obtained sponge was crosslinked by irradiating ultraviolet ray. This sponge was washed by rinsing in 1 mol/L sodium hydroxide aqueous solution, again, the freeze and the lyophilization were performed to obtain the chitosan/atelocollagen (chitosan : atelocollagen (weight ratio)= 75 : 25) sponge (a spongy molding of the present invention).

### (Observation by scanning electron microscopy)

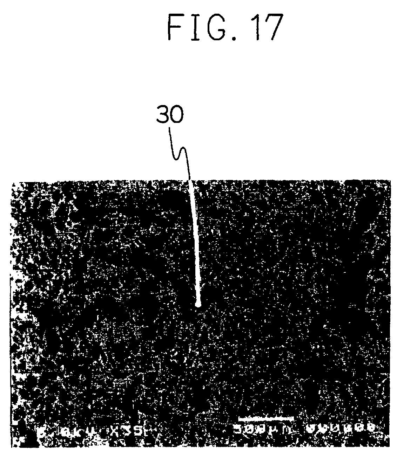

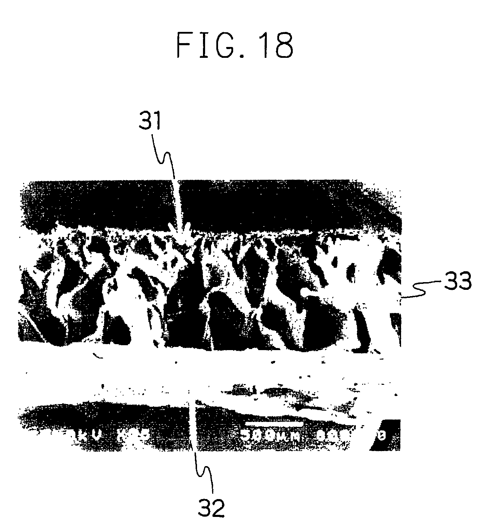

The surface and the cross section of the obtained chitosan/atelocollagen sponge were observed with the scanning electron microscope. Fig. 17 is the scanning electron micrograph (original magnification × 35) of the surface of the obtained chitosan/atelocollagen sponge and Fig. 18 is the scanning electron micrograph (original magnification × 35) of the cross section of the obtained chitosan/atelocollagen sponge.

As shown in Fig. 18, the vertically directional pores 33 penetrated from the surface 31 of sponge to near the rear surface 32 were formed, and the pores 30 having a diameter of 200-300 µm were uniformly formed in the surface 31 (refer to Fig. 17).

### EXAMPLE 7

### (Preparation of chitosan solution)

About 2 g of chitosan (derived from crab shell) was added to about 98 g of ultra pure water and was gently stirred at room temperature. With stirring, thereto 1 ml of acetic acid was added and the stirring was continued for about 4 hours at room temperature to obtain a chitosan solution.

### (Preparation of chitosan sponge)

About 20 g of the above-mentioned chitosan solution was poured into a plastic case having a size of about 100 × 100 × 20 mm. Freezing the solution was carried out by inserting the plastic case in the heat insulation frame made from polystyrene foam as the same in Example 1, putting whole heat insulation frame into the ultra-deep freezer, and then lyophilization was carried out. This sponge was washed by rinsing in 1 mol/L sodium hydroxide aqueous solution, again, the freeze and the lyophilization were performed to obtain a chitosan sponge (a spongy molding of the present invention).

### (Observation by scanning electron microscopy)

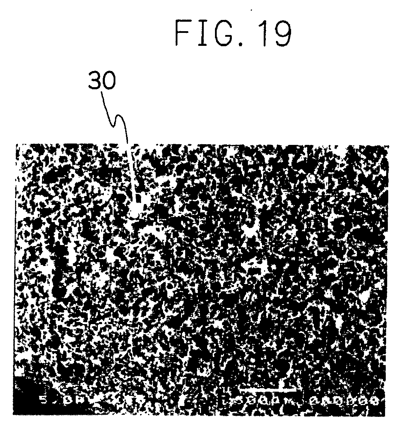

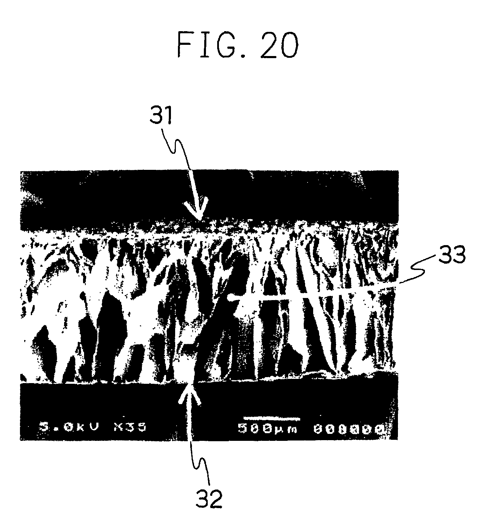

The surface and the cross section of the obtained chitosan sponge were observed with the scanning electron microscope. Fig. 19 is the scanning electron micrograph (original magnification × 35) of the surface of the obtained chitosan sponge and Fig. 20 is the scanning electron micrograph (original magnification × 35) of the cross section of the obtained chitosan sponge.

As shown in Fig. 20, the vertically directional pores 33 penetrated from the surface 31 of sponge to near the rear surface 32 were formed, and the pores 30 having a diameter of 200-300 µm were uniformly formed in the surface 31 (refer to Fig. 19).

### COMPARATIVE EXAMPLE 1

### (Preparation of atelocollagen sponge)

About 20 g of 1.0 % atelocollagen/citric acid aqueous solution (derived from the skin of adult pig) was poured into a plastic case having a size of about 100 × 100 × 20 mm, and was gelated in an atmosphere of ammonia gas. Freezing the gel was carried out by putting it into the ultra-deep freezer and then lyophilization was carried out. The obtained sponge was crosslinked by irradiating ultraviolet ray. Thereafter, this sponge was washed with water. Again, the freeze and the lyophilization were performed to obtain an atelocollagen sponge.

### (Observation by scanning electron microscopy)

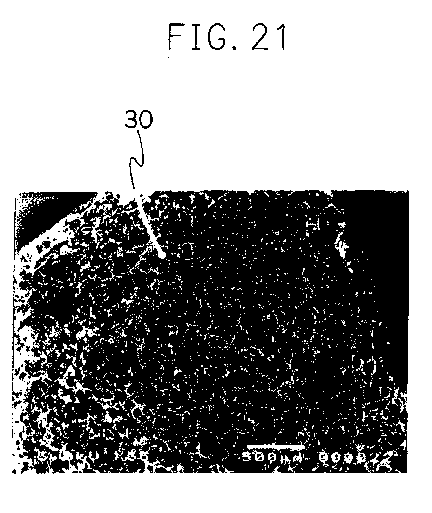

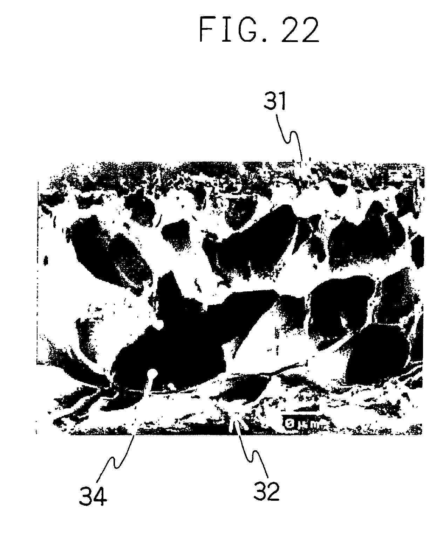

The surface and the cross section of the obtained atelocollagen sponge were observed with the scanning electron microscope. Fig. 21 is the scanning electron micrograph (original magnification × 35) of the surface of the obtained atelocollagen sponge and Fig. 22 is the scanning electron micrograph (original magnification × 35) of the cross section of the obtained atelocollagen sponge.

As shown in Fig. 22, the scanning electron micrograph of the cross section of the sponge show that independent pores 34 not communicated to either the surface 31 or to the rear surface 32 were formed within the sponge. The pores 30 having a diameter of several 10 µm is observed in the surface 31 (refer to Fig. 21).

### COMPARATIVE EXAMPLE 2

### (Preparation of chitosan solution)

A chitosan solution was prepared in the same manner as described in Example 2.

### (Preparation of chitosan/atelocollagen sponge)

About 20 g of the above-mentioned chitosan solution was poured into a plastic case having a size of about 100 × 100 × 20 mm. Freezing the solution was carried out by putting it into the ultra-deep freezer, and then lyophilization was carried out. The obtained sponge was washed by rinsing in 1 mol/L sodium hydroxide aqueous solution, again, the freeze and the lyophilization were performed to obtain a chitosan sponge.

### (Observation by scanning electron microscopy)

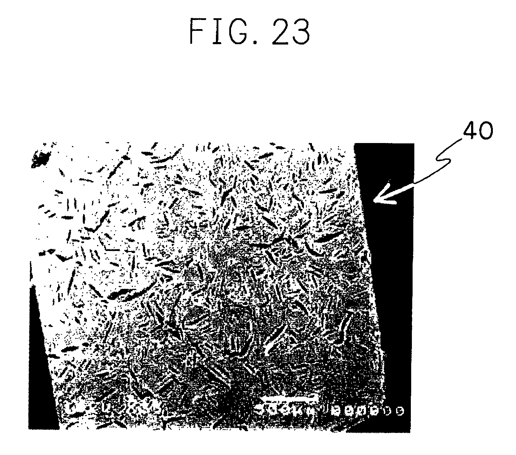

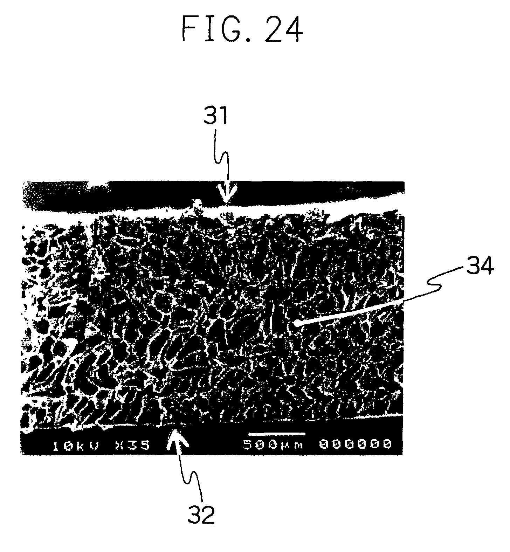

The surface and the cross section of the obtained chitosan sponge were observed with the scanning electron microscope. Fig. 23 is the scanning electron micrograph (original magnification × 35) of the surface of the obtained chitosan sponge and Fig. 24 is the scanning electron micrograph (original magnification × 35) of the cross section of the obtained chitosan sponge.

As shown in Fig. 24, the scanning electron micrograph of the cross section of the sponge show that independent pores 34 not communicated to either the surface 31 or to the rear surface 32 were formed within the sponge. Although Fig. 23 shows the surface of sponge 40, a pore was hardly observed in the surface of sponge 40, but the surface was in the film form.

### TEST EXAMPLE

### (Evaluation of infiltration of fetal bovine serum (FBS))

A vial with a bore of about 5 mm was filled with the fetal bovine serum (FBS) until the meniscus became convex.

The atelocollagen sponge having the vertically directional pores obtained in Example 1, the chitosan/atelocollagen (chitosan : atelocollagen (weight ratio) = 25 : 75) sponge having the vertically directional pores obtained in Example 2 and the atelocollagen sponge having the independent pores obtained in Comparative Example 1 was cut down so that a diameter is set to 20 mm, respectively, and Kimmwipe (trade name, available form CRECIA Corporation) was stuck on the rear surface.

The face (surface) which had not stuck Kimmwipe was turned down and the above-mentioned three kinds of sponge were delicately put on the mouth of the vial, respectively. FBS was made to permeate each sponge, and time (reaching time of FBS to rear surface) until Kimmwipe stuck on the rear surface gets wet after placing each sponge was recorded. The result is shown in Table 1. In addition, in Table 1, the evaluation result based on the following evaluation criteria was indicated.

### (Evaluation criteria)

ⓞ: Reaching time was less than 30 seconds.
○: Reaching time was at least 30 seconds and less than 1 minute.
Δ: Reaching time was at least 1 minute and less than 5 minutes.
×: Reaching time was at least 5 minutes, or FBS did not reach.

**TABLE 1**

| The number of Example | Reaching time of FBS to rear surface |
|---|---|
| 1 | ⓞ |
| 2 | ⓞ |
| Comparative Example 1 | × |

The result shown in Table 1 shows that the sponges of Examples 1 and 2 exhibit the FBS permeability which was extremely excellent as compared with the sponge of comparative Example 1. It is thought that it is because that in the sponge of Examples 1 and 2 in which the vertically directional pores is formed, FBS was absorbed at an early stage by the capillarity phenomenon to the rear surface of sponge. Moreover, it is thought that the pore walls prevented the permeation of FBS in case of the sponge of Comparative Example 1 with which the independent pores were formed.

### EXAMPLE 8

### (Preparation of atelocollagen sponge)

About 20 g of 1.0 % atelocollagen/citric acid aqueous solution was poured into a container 1a having a size of about 100 × 100 × 20 mm (in which the upper part is opened, the side surface and lower surface consist of polystyrene form 2a and stainless steel plate 50, respectively (refer to Fig. 5)), and was gelated in an atmosphere of ammonia gas. Freezing the gel was carried out by inserting the container 1a in the heat insulation frame 2b made from polystyrene foam (having a size of about 200 × 200 × 70 mm, and in the lower part thereof, the hole in which the above-mentioned plastics case can be inserted was open (refer to Fig. 6)) so that the open part might be closed, putting whole heat insulation frame 2b into a ultra-deep freezer, and then lyophilization was carried out. The bridge was constructed over the obtained sponge by irradiating ultraviolet ray. This sponge was washed with water. Again, the freeze and the lyophilization were performed to obtain an atelocollagen sponge (a spongy molding of the present invention).

### (Observation by scanning electron microscopy)

The surface and the cross section of the obtained atelocollagen sponge were observed with the scanning electron microscope.

### EXAMPLE 9

### (Preparation of atelocollagen sponge)

About 20 g of 1.0 % atelocollagen/citric acid aqueous solution (derived from the skin of adult pig) was poured into the container 1a having the size of about 100 × 100 × 20 mm (in which the upper part is opened, the side surface and lower surface consist of polystyrene form 2a and plastic plate 50 having a thickness of 0.4 mm, respectively (refer to Fig. 5)), and was gelated in an atmosphere of ammonia gas. Freezing the gel was carried out by inserting the container 1a in the heat insulation frame 2b made from polystyrene foam (having a size of about 200 × 200 × 70 mm, and in the lower part thereof, the hole in which the above-mentioned plastics case can be inserted was open (refer to Fig. 6)) so that the open part might be closed, putting whole heat insulation frame 2b into a ultra-deep freezer, and then lyophilization was carried out. The obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

The surface and the cross section of the obtained atelocollagen sponge were observed with the scanning electron microscope.

### EXAMPLE 10

### (Preparation of atelocollagen sponge)

About 20 g of 1.0 % atelocollagen/citric acid aqueous solution (derived from the skin of adult pig) was poured into a plastic case having a size of about 100 × 100 × 20 mm, and was gelated in an atmosphere of ammonia gas. The gel was froze by contacting a piece of metal which has maintained at about -50°C to the interface of the gel (contact surface of piece of metal: about 95 × 95 mm), and then the lyophilization was carried out. The obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

The surface and the cross section of the obtained atelocollagen sponge were observed with the scanning electron microscope.

### EXAMPLE 11

### (Preparation of atelocollagen sponge)

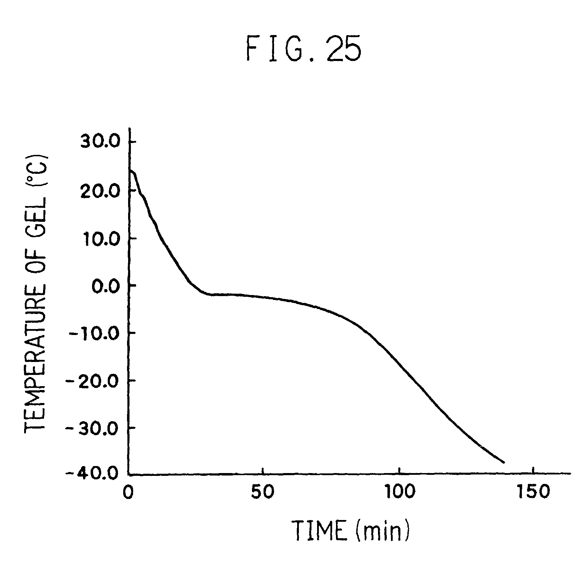

About 20 g of 1.0 % atelocollagen/citric acid aqueous solution (derived from the skin of adult pig) was poured into a plastic case having a size of about 100 × 100 × 20 mm, and was gelated in an atmosphere of ammonia gas. The plastic case was inserted in the heat insulation frame made from polystyrene foam (having a size of about 200 × 200 × 70 mm, and in the upper part thereof, the hole in which the above-mentioned plastic case can be inserted was open), and whole heat insulation frame was put into the ultra-deep freezer (setting temperature: -60°C), and the gel was frozen. The temperature history of the gel at this time is shown in the graph of Fig. 25. The cooling rate of the gel was -0.0 to -0.9°C/min. The lyophilization was carried out, and then the obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

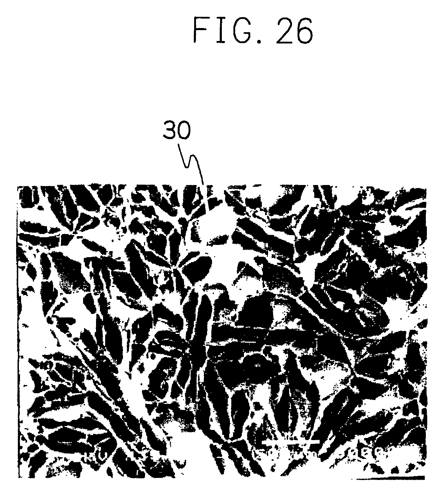

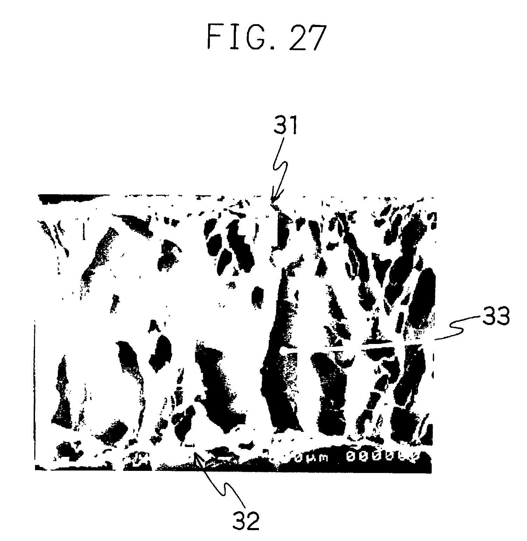

The surface and the cross section of the obtained atelocollagen sponge were observed with the scanning electron microscope. Fig. 26 is the scanning electron micrograph (original magnification × 35) of the surface of the obtained chitosan sponge and Fig. 27 is the scanning electron micrograph (original magnification × 35) of the cross section of the obtained chitosan sponge.

As shown in Fig. 27, the vertically directional pores 33 penetrated from the surface 31 to near the rear surface 32 of sponge were formed, and the pores 30 having a diameter of 10-800 µm were uniformly formed in the surface 31 (refer to Fig. 26).

### EXAMPLE 12

### (Preparation of atelocollagen sponge)

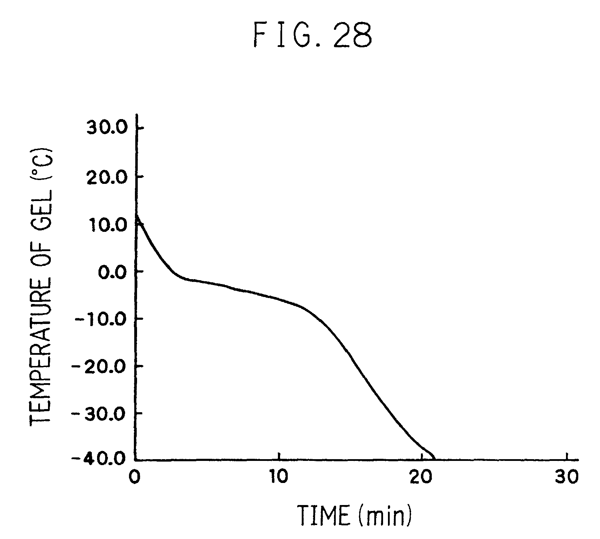

About 20 g of 1.0 % atelocollagen/citric acid aqueous solution (derived from the skin of adult pig) was poured into a plastic case having a size of about 100 × 100 × 20 mm, and was gelated in an atmosphere of ammonia gas. The plastic case was inserted in the heat insulation frame made from polystyrene foam (having a size of about 200 × 200 × 70 mm, and in the upper part thereof, the hole in which the above-mentioned plastic case can be inserted was open), and whole heat insulation frame was put into the ultra-deep freezer (setting temperature: -60°C), and the gel was frozen. The temperature history of the gel at this time is shown in the graph of Fig. 28. The cooling rate of the gel was -0.6 to -4.2°C/min. The lyophilization was carried out, and then the obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

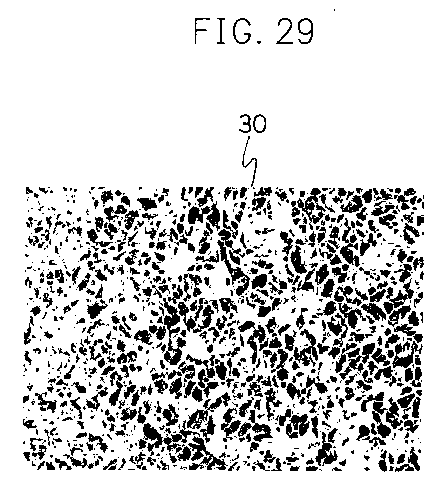

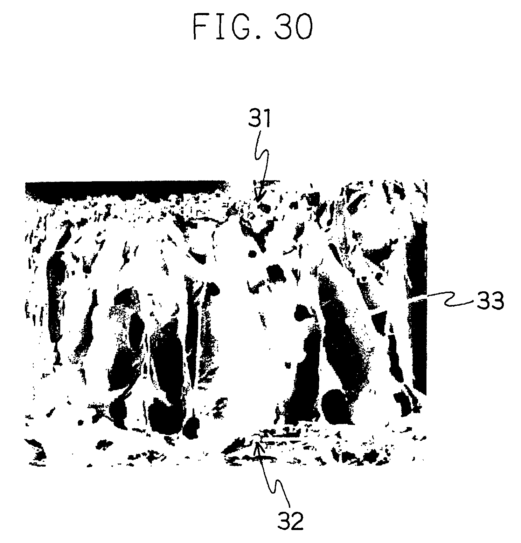

The surface and the cross section of the obtained atelocollagen sponge were observed with the scanning electron microscope. Fig. 29 is the scanning electron micrograph (original magnification × 35) of the surface of the obtained chitosan sponge and Fig. 30 is the scanning electron micrograph (original magnification × 35) of the cross section of the obtained chitosan sponge.

As shown in Fig. 30, the vertically directional pores 33 penetrated from the surface 31 to near the rear surface 32 of sponge were formed, and the pores 30 having a diameter of 10-200 µm were uniformly formed in the surface 31 (refer to Fig. 29).

### EXAMPLE 13

### (Preparation of atelocollagen sponge)

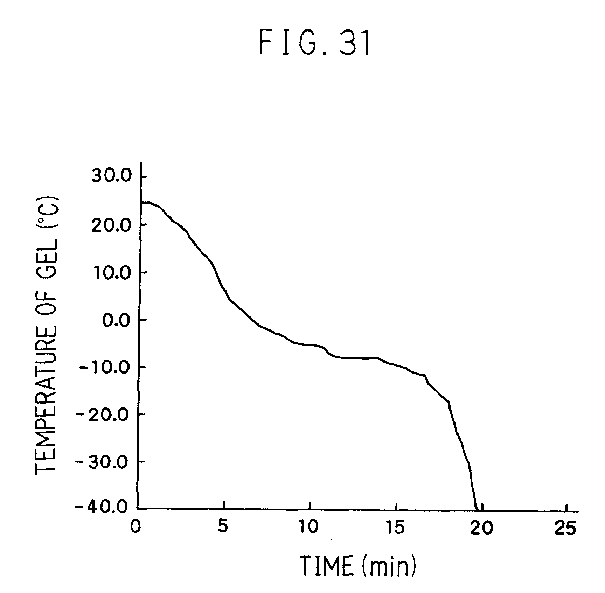

About 20 g of 1.0 % atelocollagen/citric acid aqueous solution (derived from the skin of adult pig) was poured into a plastic case having a size of about 100 × 100 × 20 mm, and was gelated in an atmosphere of ammonia gas. The plastic case was inserted in the heat insulation frame made from polystyrene foam (having a size of about 200 × 200 × 70 mm, and in the upper part thereof, the hole in which the above-mentioned plastic case can be inserted was open), and the inlet of the aperture was closed with the polystyrene form plate (thickness: 10 mm). Liquid nitrogen (about -200°C) was poured on the polystyrene form plate and the gel was frozen under this cooled atmosphere with liquid nitrogen. The temperature history of the gel at this time is shown in the graph of Fig. 31. The cooling rate of the gel was -0.0 to -31°C/min. The lyophilization was carried out, and then the obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

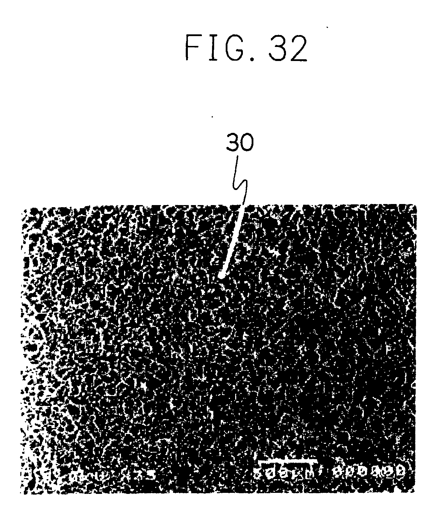

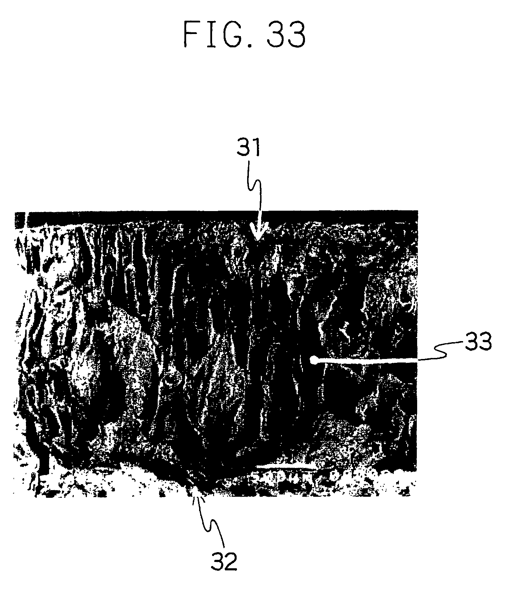

The surface and the cross section of the obtained atelocollagen sponge were observed with the scanning electron microscope. Fig. 32 is the scanning electron micrograph (original magnification × 35) of the surface of the obtained chitosan sponge and Fig. 33 is the scanning electron micrograph (original magnification × 35) of the cross section of the obtained chitosan sponge.

As shown in Fig. 33, the vertically directional pores 33 penetrated from the surface 31 to near the rear surface 32 of sponge were formed, and the pores 30 having a diameter of several 10 µm were uniformly formed in the surface 31 (refer to Fig. 32).

### EXAMPLE 14

### (Preparation of atelocollagen sponge)

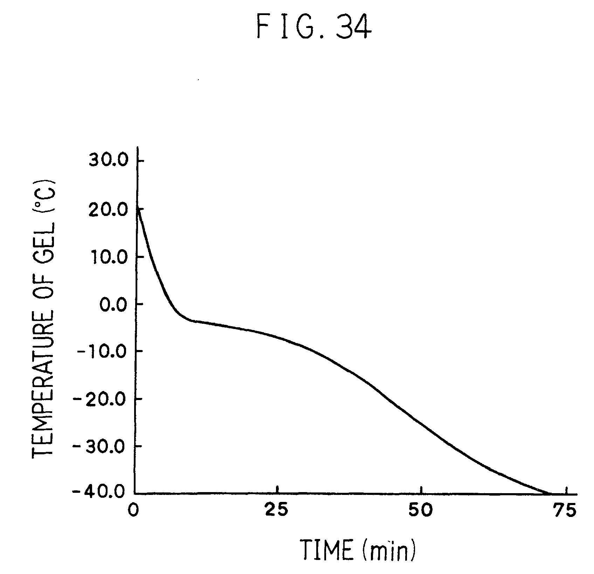

About 20 g of 1.0 % atelocollagen/citric acid aqueous solution (derived from the skin of adult pig) was poured into a plastic case having a size of about 100 × 100 × 20 mm, and was gelated in an atmosphere of ammonia gas. The plastic case was inserted in the heat insulation frame made from polystyrene foam (having a size of about 200 × 200 × 70 mm, and in the upper part thereof, the hole in which the above-mentioned plastic case can be inserted was open), and whole heat insulation frame was put into a lyophilizer (shelf temperature: to maintain at -50°C), and the gel was frozen. The temperature history of the gel at this time is shown in the graph of Fig. 34. The cooling rate of the gel was -0.2 to -3.2°C/min. The lyophilization was carried out, and then the obtained sponge was crosslinked by irradiating ultraviolet ray. This sponge was washed with water. Again, the freeze and the lyophilization were performed to obtain an atelocollagen sponge (a spongy molding of the present invention).

### (Observation by scanning electron microscopy)

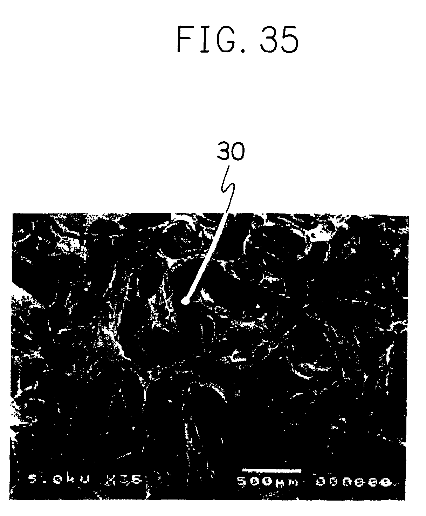

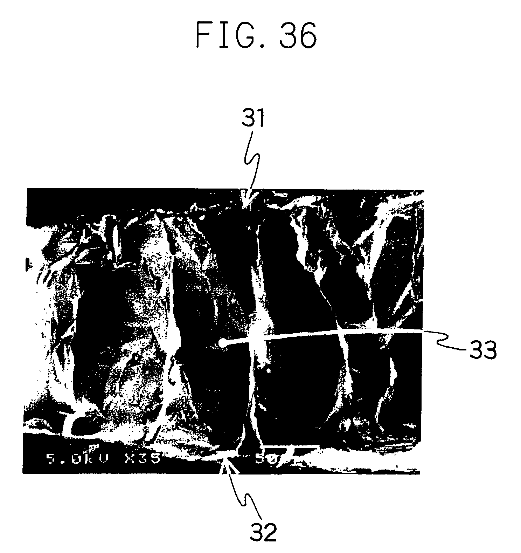

The surface and the cross section of the obtained atelocollagen sponge were observed with the scanning electron microscope. Fig. 35 is the scanning electron micrograph (original magnification × 35) of the surface of the obtained chitosan sponge and Fig. 36 is the scanning electron micrograph (original magnification × 35) of the cross section of the obtained chitosan sponge.

As shown in Fig. 36, the vertically directional pores 33 penetrated from the surface 31 to near the rear surface 32 of sponge were formed, and the pores 30 having a diameter of 10-1000 µm were uniformly formed in the surface 31 (refer to Fig. 35).

### INDUSTRIAL APPLICABILITY

According to the method for controlling pores of the present invention, a spongy molding which has plurality of vertically directional pores (in parallel with the direction of thickness of the spongy molding), with which pores having a constant size are formed in one surface, if necessary, with which a pore is hardly formed in the opposite face as to the surface and the opposite face may be substantially closed can be easily prepared. The spongy molding is extremely suitable for a wound dressing, a graft for wound (especially a graft for dermal defect) and a cultured skin matrix.

## Claims

1. A method for controlling pores of a spongy molding comprising a water-soluble polymeric material, which comprises
(a) a prefreezing step **characterized in that** a solution or a gel of the water-soluble material is prefreezed by cooling an interfacial side of the solution or the gel and air layer to induce a temperature slope in parallel with a direction of thickness within the solution or the gel, and
(b) a step of lyophilizing a solution or a gel of the water-soluble material prefreezed in according to the prefreezing step (a).

2. A method for controlling pores of a spongy molding comprising a water-soluble polymeric material, which comprises
(a) a prefreezing step **characterized in that** a solution or a gel of the water-soluble material is prefreezed by cooling an intefacial side of the solution or the gel and air layer prior to other surfaces excluding the interface side to induce a temperature slope in parallel with a direction of thickness within the solution or the gel,
(b) a step of lyophilizing the solution or the gel of the water-soluble material prefreezed in according to the prefreezing step (a).

3. The method for controlling pores of Claim 1 or 2, wherein prefreezing step (a) is performed in the state where other surfaces excluding the interfacial side of the solution or the gel of water-soluble polymeric material are covered with the heat insulating material.

4. The method for controlling pores of Claim 1 or 2, wherein prefreezing step (a) is performed by cooling the piece of a metal prepared so that an interface of a solution or a gel of water-soluble polymeric material be made to contact directly or indirectly.

5. The method for controlling pores of Claim 1 or 2, wherein prefreezing step (a) is performed by bringing the piece of a metal cooled beforehand into contact with an interface of a solution or a gel of water-soluble polymeric material, directly or indirectly.

6. The method for controlling pores of Claim 1 or 2, wherein a water-soluble polymeric material is at least one selected from a group consisting of polyacrylamide, polyvinyl alcohol, polyethylene glycol, water-soluble cellulose derivative, collagen, atelocollagen, hyaluronic acid, gelatin, chitin and chitosan.

7. The method for controlling pores of Claim 1 or 2, wherein a water-soluble polymeric material is at least one tissue-derived biomaterial selected from a group consisting of collagen, atelocollagen, hyaluronic acid, gelatin, chitin and chitosan.

8. The method for controlling pores of Claim 1 or 2, wherein a water-soluble polymeric material atelocollagen and/or chitosan.

9. The method for controlling pores of Claim 1 or 2, wherein a spongy molding is a wound dressing or a graft for wound.

10. The method for controlling pores of Claim 1 or 2, wherein a spongy molding is a cultured skin matrix.

11. A spongy molding prepared according to the method for controlling pores of Claim 1, which has plurality of vertically directional pores in parallel with the direction of thickness, in which said plurality of vertically directional pores are opened in a surface which was said interface and/or the opposite surface as to said surface which was the interface.

12. The spongy molding of Claim 11, which is a wound dressing or a graft for wound.

13. The spongy molding of Claim 11, which is a cultured skin matrix.

14. a spongy molding prepared according to the method for controlling pores of Claim 2, which has plurality of vertically directional pores in parallel with the direction of thickness, in which said plurality of vertically directional pores are opened in a surface which was said interface and/or the opposite surface as to said surface which was the interface.

15. The spongy molding of Claim 14, which is a wound dressing or a graft for wound.

16. The spongy molding of Claim 14, which is a cultured skin matrix.
